# EUROPEAN PATENT APPLICATION

(11) **EP 4 682 248 A1**
(43) Date of publication of application: **21.01.2026**
(21) Application number: 24774051.7
(22) Date of filing: 17.03.2024
(51) Int. Cl.: C12N 5/071, C12N 5/09, C12Q 1/02

(54) **NEW HUMAN TISSUE ORGANOID CULTURE SYSTEM**

(30) Priority: 17.03.2023 WO PCT/CN2023/082192
(71) Applicant: Peking University, Beijing 100871 (CN)
(72) Inventor: DENG, Hongkui, Beijing 100871 (CN); QU, Molong, Beijing 100871 (CN); XIONG, Liang, Beijing 100871 (CN)
(74) Representative: Altmann Stößel Dick Patentanwälte PartG mbB
(86) International application number: PCT/CN2024/082075
(87) International publication number: WO 2024/193486

(57) **Abstract**

Provided is a composition for obtaining and/or culturing the human normal tissue derived organoids or the human tumor tissue derived organoids, the composition includes an ALK inhibitor (*e.g.*, LDN214117), a WNT agonist (*e.g.*, CHIR), an EGF agonist (*e.g.*, EGF) and an FGF agonist (*e.g.*, FGF2), and optionally an IKKε inhibitor.

## Description

### FIELD OF THE INVENTION

The present invention relates to the human tissue organoids, such as the organoids derived from the human normal tissues (such as the intestinal tissues) and various tumor tissues such as the colorectal tumor tissues, and particularly to the human intestinal organoids and the colorectal tumor organoids, the uses thereof and the preparation methods thereof.

### BACKGROUND OF THE INVENTION

The development of organoid technology allows *in vitro* self-organization of the stem cells, thereby simulating the complex structures and functions of *in vivo* tissues. Organoid culture technology has been developed on a variety of tissues and organs, which provides a valuable tool for the study of differentiation and development and disease treatment. However, the existing human organoid culture technologies have difficulties in capturing the characteristics of in situ normal tissues and organs and the tumor tissues, and the expansion rate of organoid is slow. These defects severely limit the clinical application of human tissue organoid technology. In order to make up for the shortcomings of the existing human tissue organoid technologies, a new human tissue organoid technology has been established herein, which can capture and maintain the organoids with characteristics similar to those of in situ normal tissues and organs such as the intestinal tissues or tumor tissues *in vitro,* and has higher expansion efficiency.

### SUMMARY OF THE INVENTION

Three-dimensional organoids have the ability to simulate the structures and functions of tissues under physiological conditions, thereby providing a valuable tool for *in vitro* modeling and disease research. However, the existing human organoid (*e.g*., the human intestinal organoid) culture technologies have difficulties in capturing the characteristics of in situ normal tissues and organs and the tumor tissues. Here, a new human organoid culture system has been established, which can capture and maintain the key characteristics of in situ normal tissues and organs or the tumor tissues. In summary, our research has established a new organoid model derived from the human normal tissues and organs and the tumor tissues, opening up a new way to explore the development of human tissues and organs and the development and progression of tumors.

In an exemplary embodiment of the present invention, a novel human intestinal organoid culture system has been established, which can capture and maintain the key characteristics of in situ intestinal tissues and colorectal tumor tissues. Compared with the conventional intestinal organoids, the organoids derived from the human intestinal tissues and colorectal tumor tissues are significantly enriched in epithelial cell heterogeneity such as the activation of goblet cell lineage genes, and the proliferation and expansion ability of organoids is significantly enhanced.

Specifically, the present invention provides a composition and a method for culturing and obtaining a novel human tissue organoid with the characteristics of in situ normal tissues and organs and tumor tissues. Specifically, the present invention provides the following technical solutions:
1. A composition comprising: (1) an ALK inhibitor, (2) a WNT agonist, (3) an EGF agonist and (4) an FGF agonist; preferably the composition is used to obtain and/or culture a human tissue organoid, for example, a human normal tissue derived organoid or a human tumor tissue derived organoid, such as a human intestinal tissue derived organoid or a human colorectal tumor tissue derived organoid.
2. The composition according to item 1, wherein the (1) ALK inhibitor is one or more selected from the group consisting of: LDN193189, LDN214117, LDN212854, ML347, and K02288; preferably LDN214117.
3. The composition according to item 1, wherein the (2) WNT agonist is one or more selected from the group consisting of: CHIR-99021, CHIR-98014, CHIR-98023, CHIR-98024, GSK3β inhibitor XV, TD114-2, and BIO-acetoxim; preferably CHIR-99021.
4. The composition according to item 1, wherein the (3) EGF agonist is one or more selected from the group consisting of: EGF, HB-EGF, AREG, EREG, NRG1, NRG2, NRG3, and NRG4; preferably EGF.
5. The composition according to item 1, wherein the (4) FGF agonist is one or more selected from the group consisting of: FGF1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, and FGF23; preferably FGF2.
6. The composition according to any one of items 1-5, further comprising (5) an IKKε inhibitor;
   optionally, the (5) IKKε inhibitor is one or more selected from the group consisting of: WS6, WS3, TBK1/IKKε-IN-5, TBK1/IKKε-IN-2, TBK1/IKKε-IN-6, TBK1/IKKε-IN-4, TBK1/IKKε-IN-1, GSK319347A, HPN-01, AY-985, Amlexanox, MRT67307, BX795, and DMX-129; preferably WS6.
7. A method for obtaining and/or culturing a human tissue organoid, comprising: culturing a human tissue in a culture medium, wherein the culture medium comprises the composition according to any one of items 1-5; preferably, the human tissue includes a human normal tissue or a human tumor tissue, and the human tissue organoid includes a human normal tissue derived organoid or a human tumor tissue derived organoid.
8. The method according to item 7, wherein the human normal tissue is a human intestinal tissue, stomach, lung, liver or ovary, and the human tumor tissue is a human colorectal cancer tumor tissue, a human gastric cancer tumor tissue, a human lung cancer tumor tissue, a human liver cancer tumor tissue or a human ovarian cancer tumor tissue.
9. The method according to any one of items 7-8, wherein DMEM/F12 is used as a basal culture medium;
   preferably, HEPES, GlutaMAX, B27, and 1 mM N-Acetylcysteine are further added to the basal culture medium.
10. A human normal tissue derived organoid or human tumor tissue derived organoid obtained by the method of any one of items 7-9.
11. The human normal tissue derived organoid or the human tumor tissue derived organoid according to item 10, which is a human intestinal tissue derived organoid or a human colorectal tumor tissue derived organoid, and wherein it expresses one or more of the marker genes LGR5, OLFM4, CHGA, CHGB, MUC2, AQP8 and LYPD8.
12. The human tumor tissue derived organoid of item 10, for use in *in vitro* identification of the drug resistance of a tumor, the metastatic and invasive ability of a tumor, for use in screening of a drug that is highly effective in inhibiting or killing a tumor cell, or for use as an *in vitro* model for reconstructing the process of tumor development.
13. A method for obtaining and/or culturing a human tumor organoid, comprising: culturing the human tumor tissue or the human tumor tissue derived organoid of item 10 in a culture medium, wherein the culture medium comprises the composition of item 6.
14. A human tumor organoid obtained according to the method of item 13, comprising at least one epithelial-mesenchymal plasticity marker gene with an increased expression level.

### TECHNICAL EFFECTS OF THE INVENTION

The organoid culture system of the present invention can be used to establish a novel human normal tissue/organ organoid or tumor tissue organoid system with the characteristics similar to those of in situ normal tissues/organs or the tumor tissues *in vivo.* The human normal tissue (such as intestinal tract) organoids obtained by the culture system of the present invention can be used to simulate the complex structures and functions of *in vivo* tissues such as the intestinal tissues, providing a valuable tool for the study of development and treatment of diseases or tumors in tissues. The tumor tissue organoids obtained by the culture system of the present invention can faithfully reflect the pathological and genomic characteristics of the primary tumor.

### EXPERIMENTAL MATERIALS AND METHODS

### 1. Obtaining Tumors and Para-carcinoma Tissues

Tumor tissues and adjacent normal tissues were obtained from the hospital surgeries, and the informed consent forms were signed and approved by the Ethics Committee. For example, the clinical data of some patients were exemplarily listed in Table 1.

**Table 1 Clinical treatment of patients**

| **Patient number** | **Tumor type** | **Location** | **Gender** | **Age** | **Pathological stage of tumor** | **Clinical stages** | **Microsatellite** |
|---|---|---|---|---|---|---|---|
| P1 | Moderately-Poorly differentiated adenocarcinoma | Ascending colon | Female | 82 | pT3N2aM0 | III | Microsatellite stable |
| P2 | Moderately differentiated adenocarcinoma | Rectum | Female | 80 | pT3N0M0 | II | Microsatellite stable |
| P3 | Poorly differentiated adenocarcinoma with mucinous adenocarcinoma | Sigmoid colon | Male | 72 | pT3N0M0 | II | Microsatellite unstable |
| P4 | Well differentiated adenocarcinoma | Ascending colon | Male | 90 | pT3N0M0 | II | Microsatellite stable |
| P5 | Moderately differentiated adenocarcinoma | Rectum | Female | 78 | pT3N0M0 | II | Microsatellite stable |
| P6 | Moderately differentiated adenocarcinoma | Rectum | Male | 59 | pT3N2bM0 | III | Microsatellite stable |
| P7 | Moderately differentiated adenocarcinoma | Descending colon | Female | 58 | pT4bN1bM0 | III | Microsatellite stable |
| P8 | Moderately differentiated adenocarcinoma | Rectum | Female | 66 | pT4aN0M0 | II | Microsatellite stable |
| P9 | Moderately differentiated adenocarcinoma | Rectum | Female | 80 | pT3N0M0 | II | Microsatellite stable |
| P10 | Micropapillary adenocarcinoma | Sigmoid colon | Female | 53 | rypT4bN2aM0 | III | Microsatellite stable |
| P11 | Moderately differentiated adenocarcinoma | Ascending colon | Male | 62 | pT3N0M0 | II | Microsatellite stable |
| P12 | Moderately differentiated adenocarcinoma | Sigmoid colon | Male | 62 | pT3N0/pT2N1bM0 | II | Microsatellite stable |
| P13 | Moderately differentiated adenocarcinoma | Rectum | Male | 57 | pT3N1b, cM1a | IV | Microsatellite stable |
| P14 | Moderately differentiated adenocarcinoma | Transverse colon | Male | 72 | pT4aN2bM1c | IV | Microsatellite stable |
| P15 | Moderately differentiated adenocarcinoma | Rectum | Male | 58 | pT3N2a, cM1a | IV | Microsatellite stable |
| P16 | Moderately differentiated adenocarcinoma | Rectum | Male | 63 | pT3N0M0 | II | Microsatellite stable |
| P17 | Moderately differentiated adenocarcinoma | Transverse colon | Female | 57 | pT3N2a, cM1c | IV | Microsatellite stable |
| P18 | Moderately-Poorly differentiated adenocarcinoma with mucinous adenocarcinoma | Sigmoid colon | Male | 59 | pT3N2bM0 | III | Microsatellite stable |
| P19 | Moderately differentiated adenocarcinoma | Rectum | Male | 77 | pT3N0M0 | II | Microsatellite stable |
| P20 | Moderately differentiated adenocarcinoma | Rectum | Male | 77 | ypT2N0M0 | I | Microsatellite stable |
| P21 | Mucinous adenocarcinoma | Ascending colon | Female | 67 | pT4aN2a | IV | Microsatellite stable |

### 2. Tissue isolation and organoid culture

Tumors and adjacent normal tissues were isolated from the surgically resected tissues and rinsed at least 10 times with pre-cooled PBS containing 2% penicillin/streptomycin (GIBCO). Tumor cells and normal tissues (*e.g*., colonic crypts or gastric tissues) were isolated and cultured as follows. Briefly, the adjacent normal tissues such as stomach or colon were cut into 2 to 4 mm fragments, incubated in 5 mM EDTA at 4°C for 30 minutes with gentle shaking, and then vigorously shaken in pre-cooled PBS to release the normal tissue parts (*e.g*., colonic crypts). Tumor tissues were minced into small pieces of 0.5 mm³, and placed in a 10 cm sterile dish. 5 ml of digestion solution containing 500 U/mL collagenase IV (Sigma-aldrich, C9407), 1.5 mg/mL collagenase II (Solarbio, C8150), 20 mg/mL hyaluronidase (Solarbio, h8030), 0.1 mg/mL diase II (Sigma-aldrich, D4693), 10 µM Y27632 and 1% fetal bovine serum was added and digested at 37°C for 30-60 minutes. Tumor cells were collected by centrifugation at 500 g for 5 minutes. Normal tissue parts (*e.g.*, colonic crypts) or tumor cells were suspended in Matrigel and distributed in 20 µL droplets on preheated 48-well plates and incubated in a 37°C, 5% CO₂ incubator for 10-15 minutes. After the Matrigel polymerization, 500 µL of Advanced DMEM/F12 (Gibco) medium containing 10 mM HEPES (Gibco), 1× GlutaMAX (Gibco), 1% penicillin/streptomycin (Gibco), 1× N2 Supplement (Gibco) (optionally), 1× B27 Supplement (Gibco), and 1.25 mM n-acetylcysteine (Sigma-Aldrich) was added.

Conventional culture conditions for the human colorectal normal tissue derived organoids are known in the prior art (see, for example, DOI: 10.1038/nature07935), and are specifically as follows: a culture medium containing 50% Wnt conditioned medium, 20% R-Spondin conditioned medium, 100 ng/ml Noggin, 50 ng/ml EGF, 10 mM Niacinamide, 10 nM Gastrin, 500 nM A83-01, 3 µM SB202190, and 10 nM prostaglandin E2.

Conventional culture conditions human colorectal tumor tissue derived organoids are known in the prior art (see, for example, DOI: 10.1038/nature07935), and are specifically as follows: a culture medium containing 20% R-Spondin conditioned medium, 100 ng/ml Noggin, 50 ng/ml EGF, 10 mM Niacinamide, 10 nM Gastrin, 500 nM A83-01, 3 µM SB202190, and 10 nM prostaglandin E2.

For the normal tissue derived organoids and the tumor tissue derived organoids (*e.g*., the intestinal tissue derived organoids and the colorectal tumor tissue derived organoids), the culture medium contains an ALK inhibitor (*e.g.*, 2 µM LDN-214177), an EGF agonist (*e.g.*, 50 ng/ml EGF), a WNT agonist (*e.g.*, 3 µM CHIR-99021), and an FGF agonist (*e.g.*, 100 ng/ml FGF2), *i.e*., a 4C condition.

The culture medium is refreshed every other day. For passage, the organoids or tumoroids were removed from Matrigel and mixed with TrypLE Express (Gibco). After incubation at 37°C for 5 minutes, the organoids or tumoroids were dissociated into small pieces, then suspended in Matrigel and distributed into the new 48-well plates. Passage was performed every 1-2 weeks (preferably, the passage ratio for the conventional organoids or tumoroids was 1:6, and the passage ratio for the intestinal tissue derived organoids or the colorectal tumor tissue derived organoids was 1:16). For a single-cell passage, the digestion time was extended to 20-30 minutes, and then the cells were filtered with a 40 µm cell strainer and suspended in Matrigel at a concentration of 4000 cells/20 µL Matrigel. It is recommended to use 10 µm Y-27632 for the first 2 days after the single-cell passage. The reagent details for organoid culture are shown in Table 2.

**Table 2 Sources of reagents**

| **Name** | **Supplier** | | | **Cat. No.** |
|---|---|---|---|---|
| LDN-214117 | | MCE | | HY-16712 |
| CHIR-99021 | | MCE | | HY-10182 |
| A 83-01 | | MCE | | HY-10432 |
| SB 202190 | | MCE | | HY-10295 |
| WS6 | | MCE | | HY-12461 |
| PD0325901 | | MCE | | HY-10254 |
| Prostaglandin E2 | | MCE | | HY-101952 |
| Nicotinamide | | MCE | | HY-B0150 |
| N-Acetylcysteine | | Sgima | | A9165 |
| Gastrin I | | R&D | | 3006 |
| Recombinant human EGF | | PeproTech | | AF-100-15 |
| Recombinant Murine Noggin | | PeproTech | | 250-38 |
| Recombinant human R-Spondin-1 | | PeproTech | | 120-10C |
| FGF2 (NM_002006) Human recombinant protein | | Origene | | TP750002 |

### 3. Immunohistochemical and immunofluorescence staining

For immunofluorescence staining, the organoids were released from Matrigel with the cell recovery solution (Coming) and fixed with 4% paraformaldehyde (DingGuo) for 15 minutes at room temperature. After the permeabilization of cells with PBS containing 0.1% Triton X-100 (Sigma-Aldrich), the primary antibody was added and incubated overnight at 4°C, followed by incubation with the secondary antibody for 1 hour at 37°C. Nuclei were stained with DAPI (Roche Life Science). Immunohistochemical staining was performed on 8 µM paraffin-embedded tissue sections after being embedded via a standard histological protocol. Heat-induced antigen retrieval was performed with Tris-EDTA (pH 9.0) buffer, and the slides were blocked with a blocking solution (PBS, 0.1% Triton X-100, 5% serum homologous to the secondary antibody) for 1 hour at room temperature. Slides were incubated with the primary antibody overnight at 4°C, followed by incubation with the secondary antibody for 1 hour at room temperature. Nuclei were stained with DAPI (Roche Life Science). Details of the antibodies used are shown in Table 3.

**Table 3 Antibody information**

| **Name** | | **Supplier** | | **Cat. No.** |
|---|---|---|---|---|
| Anti-OLFM4 protein antibody | | abcam | | ab96280 |
| Anti-MUC2 protein antibody | | abcam | | ab11197 |
| Anti-Chromogranin A protein antibody | | abcam | | ab45179 |
| Anti-Cytokeratin 20 protein antibody | | abcam | | ab854 |
| Anti-Ki67 protein antibody | | abcam | | ab15580 |
| Anti-EpCAM protein antibody | | abcam | | ab223582 |
| Anti-CDX2 protein antibody | | Biogenex | | MU392A-UC |
| Anti-β-catenin protein antibody | | BD Biosciences | | 610153 |
| Anti-Villin protein antibody | | abcam | | ab201989 |
| Anti-ACE2 protein antibody | | R&D | | AF933-SP |
| Anti-HNF4A protein antibody | | abcam | | ab41898 |
| Anti-CK7 protein antibody | | Invitrogen | | MAS-13156 |
| Anti-CK19 protein antibody | | Invitrogen | | MA5-12663 |
| PE-conjugated anti-human CD26 antibody | | Biolegend | | 302705 |
| 647 fluorophore-labeled anti-goat IgG (H+L) antibody | Jackson ImmunoResearch Laboratories | | | 705-605-147 |
| Cy^{™}3 fluorophore-labeled anti-rabbit IgG (H+L) antibody | Jackson ImmunoResearch Laboratories | | | 711-165-152 |
| Cy^{™}3 fluorophore-labeled antimouse IgG (H+L) antibody | Jackson ImmunoResearch Laboratories | | | 715-165-150 |

### 4. Renal capsule transplantation

One million tumor cells were resuspended in 50 µl of Matrigel and injected into the lower pole of the kidney (below the renal capsule) of 6-week-old male NPG mice (purchased from Beijing Vitalstar Biotechnology Co., Ltd.). The needle was removed after the blister formation was observed and the tumor cell suspension was almost no longer leaking. The clearly visible blisters were formed in the animals used in the study with very small amount of the fluid leakage when the capsule was injected. Mice were sacrificed 6 weeks after transplantation. The whole kidney was fixed with 10% and 20% formalin for 24 hours at room temperature. After the paraffin embedding, a 5 µm section was cut and mounted on the slide for immunohistochemical staining. Hematoxylin-eosin (H&E) staining was used for tissue and cell identification.

### 5. Global RNA sequencing analysis

Global RNA sequencing count data were transferred to transcripts per million (TPM) and log-normalized, and corrected for batch effects by the ComBat_seq function. Hclust was used to cluster the samples and calculate the distances. The differentially expressed genes (DE genes) were obtained using the DESeq2 package (v1.34.0) and displayed in volcano plots and heat maps, respectively. Gene set enrichment analysis (GSEA) was performed for DE genes. Gene Set Variation Analysis (GSVA) was performed for 11 different EMT-related features to find the differential expression of these EMT-related features in the samples based on the log-TPM of these epithelial-mesenchymal plasticity-related features using the GSVA R package. GSVA scores were visualized using the heatmaps and the boxplots.

### 6. Single-cell RNA sequencing analysis

Sequencing data were processed by DNBelab_C_Series_HT_scRNA-analysis-software. Cells were filtered based on the number of transcripts and the percentage of mitochondrial transcripts. An expression matrice was converted to a Seurat object using the Seurat package (v4.1.1). The scRNA-seq count data of 49,155 epithelial cells in the iCMS dataset were reanalyzed. Consensus non-negative matrix factorization (cNMF) was performed using the cNMF package and 6,000 highly variable genes. K of 6 and the density threshold of 0.1 were selected based on stability and error. Unsupervised clustering (resolution = 0.2) was performed using these 6 cNMF components to obtain 10 cell states. Marker genes for each state were found by FindAllMarkers, and the expression levels of marker genes relating to pan-cancer epithelial-mesenchymal plasticity were determined by AddModuleScore_UCell. UMAP analysis was performed based on cNMF using the UMAP-learn method. The scRNA-seq data of the conventional tumor organoids and the colorectal tumor tissue derived organoids were combined after quality filtering and logarithmic normalization. The combination of 12,718 cell data was processed by FindVariableFeatures, ScaleData, and RunPCA, with all parameters as default parameters. Then, the batch effects arising from the two different culture conditions were eliminated using the RunHarmony function, and the top 50 dimensions of PCA were used.

### 7. Cell lineage classification

Cells from in situ tumor tissue, the conventional tumor organoids, and the colorectal tumor tissue derived organoids were made into the Seurat clusters. The batch effects were eliminated using the RunHarmony function of package Harmony, and then the RunUMAP and FindNeighbors analysis was performed via Harmony at a 1:20 dimension. Cells were first classified into the epithelial and non-epithelial cells based on the known characteristics of the epithelial cells using the gating_model function of the scGate (v1.4.1) package. 20,223 epithelial cells were further clustered to analyze the differentially expressed genes with the FindAllMarkers function. Ten cell types were annotated by comparing the markers with the known epithelial lineage markers. The cell types and major markers were as follows: the stem cells (LGR5, SMOC2, ASCL2, OLFM4); the rapidly expanding cells_1 (MKI67, TOP2A, HIST1H4C, and CENPF); the rapidly expanding cells_2 (HILPDA, LGALS1, and RBP1); the intestinal progenitor cells (PYCARD); the enterocytes (EPCAM, FABP1, PHGR1, and CA2); the mature enterocytes (SLC26A3, SLC26A3, and AQP8); the secretory precursor cells (FCGBP and TFF3); the goblet cells (FCGBP, TFF3, MUC2, and SPINK4); the tuft cells (LRMP and BMX); and the enteroendocrine cells (CHGA, CHGB, SCGN, and FEV).

### 8. Whole exome sequencing analysis

Sequencing was performed using the maximum exact match alignment (BWA-MEM) v0.7.8-r455 and aligned to the human reference genome GRCh37. According to the best practice guidelines, the bam files were processed using the Genome Analysis Toolkit (GATK) v3.8.0, including marking the duplicates, reindexing, and the base recalibration. The reference data and the tumor or organoid sequencing data were provided to MuTect2 (involving GATK v3.8.0), and the somatic mutations were identified using the default parameters. By using anno var, the effect predictions and annotations were added. For high-quality detection of the somatic CNAs, the read depth variations in BAM files were interpreted by Control-FREEC v11.4, and the tumors or organoids were compared to the reference cells. Mutational signatures were analyzed using the Mutational Patterns R package v3.4 to identify the genomes of all somatic SNVs and determine the contribution of the "signature of the human cancer mutational process" in in situ tumors and the tumor organoid samples.

### 9. CNV analysis

CNV inference using the single-cell transcriptome data was based on a previously published method modified by the inferCNV software. Only the genes with an average relative expression of more than 1.5 in a single cell and passing all quality controls were used for the CNV inference. Firstly, the CNV score of one gene was reduced to the average expression level of its 100 neighboring genes. Then, the CNV scores were aggregated to zero by subtracting the average CNV score of all cells. Finally, the relative CNV score was calculated by the CNV scores of all genes within an average 10M CNV window.

### 10. Tumor purity prediction

To calculate tumor purity, inferCNV was applied to estimate copy number from single-cell transcriptome data. Inferred ecnv analysis identified 6 groups of cells with different copy number variation patterns in *in vivo* tumor tissues. Among the 6 groups, no copy number variation was found in all chromosomes of group 6, and the cells in group 6 were considered to be non-malignant cells. Following the same strategy, group 6 of the tumor organoids cultured under the conventional conditions and group 8 of the colorectal tumor tissue derived organoids were considered to be non-malignant cells, and the remaining cells were considered to be malignant cells. The tumor purity was then determined by calculating the ratio of the number of malignant cells to the total number of cells in each sample. Based on the above method, the proportions of the malignant cells to the total cells in *in vivo* tumor tissues, the conventional tumor organoids, and the colorectal tumor tissue derived organoids were finally found to be 87%, 90% and 94.2%, respectively.

### 11. Statistical analysis

All values are expressed as mean ± SEM. Statistical parameters, including statistical analysis methods, statistical significance thresholds, and n values are described in the legends of the figures and the supplementary figures.

### 12. Data availability

All data generated in the current study can be obtained from the GEO database. All other data used in this study are provided as source data herein.

### 13. Detection of the tumor invasiveness

The metastatic invasiveness of tumor cells was detected using a permeable cell culture chamber (Transwell^{®}). The operation steps were as follows: Firstly, 1.5 mg/ml Matrigel was coated on the upper chamber surface of the bottom membrane of the Transwell chamber, and incubated in a 37°C incubator for 1-4 hours to allow Matrigel to polymerize into a gel; then, the tumor cells were seeded on the upper chamber surface of the bottom membrane of the Transwell chamber at about 1 × 10⁵ cells/chamber, and 20% serum was added to the culture medium under the bottom membrane of the Transwell chamber. After culturing for 2-3 days, the Transwell chamber was removed, washed twice with PBS, fixed with methanol for 30 minutes, and stained with 0.1% crystal violet for the subsequent counting and statistics.

### 14. In vivo transplantation model of tumor organoids in mice

NOG mice (NOD.Cg-PrkdcscidII2rgtmISug/JicCrl) aged 6-8 weeks and weighing 18-20 g were purchased from Beijing Charles River Laboratory Animal Technology Co., Ltd. (Beijing, China), wherein the genders of the mice used were consistent with those of the sources of the transplanted tumor organoids. Mice were anesthetized with Avertin (JT 0781, Beijing Jitian Biotechnology Co., Ltd.) before surgery. Approximately 2 × 10⁶ cells were suspended in the Advanced DMEM/F12 (Gibco), mixed with Matrigel (BD Biosciences) in a 1:1 ratio, and injected into the subcapsular of the spleen in a final volume of 50 µL. After injection, the spleen was gently returned to the peritoneal cavity to restore the homeostasis.

*In vivo* bioluminescence imaging was performed using the *in vivo* imaging system (IVIS) Spectrum CT (PerkinElmer), and the tumor burden was quantified in a blinded manner. Regions of interest (ROIs) were created and measured as regional fluxes, and defined as the average radiation (photons/second/cm²/stereoscopic surface). Data analysis was performed using Living Image V.4.0 software (Caliper Life Sciences). All animal experiments were performed in accordance with NIH guidelines. All procedures involving mice were approved by the Institutional Animal Ethics Committee of Peking University and the Laboratory Animal Department of Beijing Cancer Hospital.

### DESCRIPTION OF THE DRAWINGS

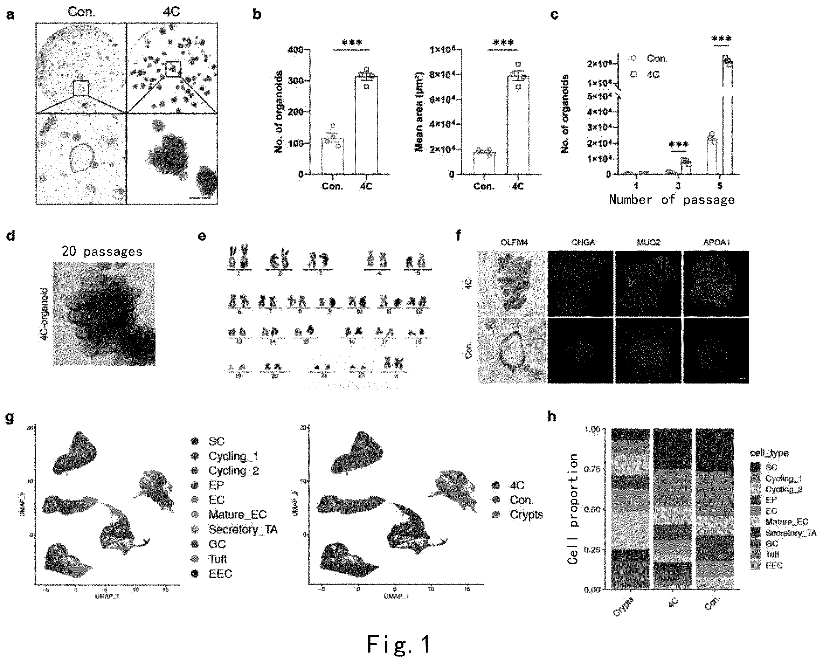

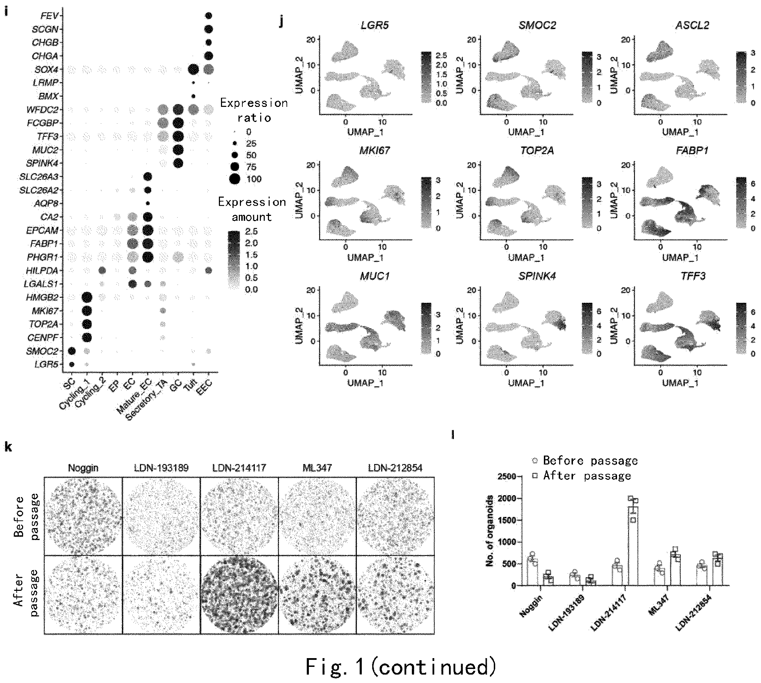

**Figure 1** **shows the establishment of intestinal tissue derived organoids.**
   a. Typical morphology of the human intestinal tissue derived organoids cultured under different conditions. Imaging was performed on day 10 after single-cell passaging.
   b. Quantification of the numbers and sizes of organoids under different conditions (n = 4).
   c. Statistics on the numbers of organoids under different conditions at different passages (n = 3).
   d. Representative images of the human intestinal tissue derived organoids under the 4C condition after the long-term passage (20 passages).
   e. Karyotype analysis of the human intestinal tissue derived organoids cultured for more than 100 days under the 4C condition.
   f. Immunohistochemical and immunofluorescence staining of various lineage cell markers in the intestinal tissue derived organoids under the 4C condition and the conventional intestinal organoids.
   g. UMAP visualization of the single-cell transcriptome data of the organoids cultured under different conditions and the intestinal crypt tissues. Left, the colors indicate unsupervised clustering. Right, the colors indicate different sample sources.
   h. The histogram shows the proportion of each cell type in the organoids cultured under different conditions and the intestinal crypt tissues.
   i. Expression of the characteristic markers for cells of different lineages.
   j. Representative gene expression of each cell type is superimposed on the UMAP shown in Panel g.
   k. Typical morphology of the human intestinal tissue derived organoids cultured with different ALK inhibitors. Imaing was performed on day 10 after single-cell passaging.
   l. Quantification of the numbers of the human intestinal tissue derived organoids cultured with different ALK inhibitors (n = 3).

   Abbreviations: Con.: the conventional culture condition of the intestinal organoid; 4C: the novel culture condition of the intestinal organoid.
   *** P < 0.001; ** P < 0.01; ** P < 0.05. Scale bar: 100 µm.

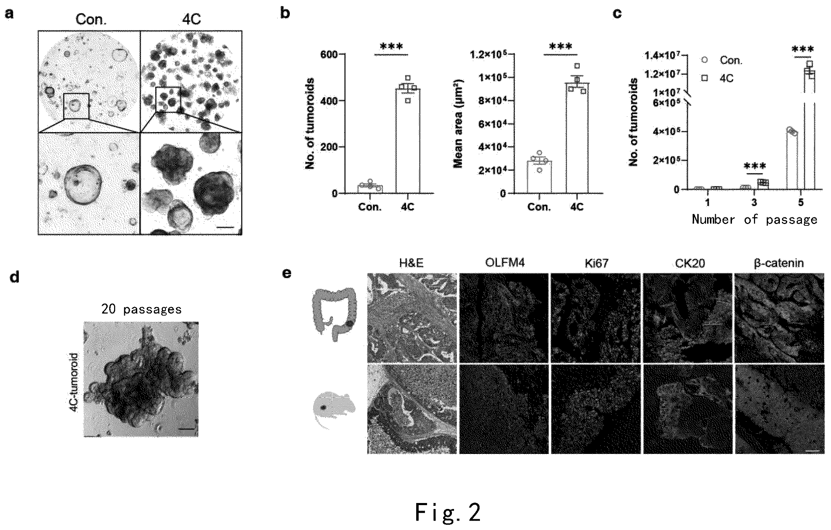

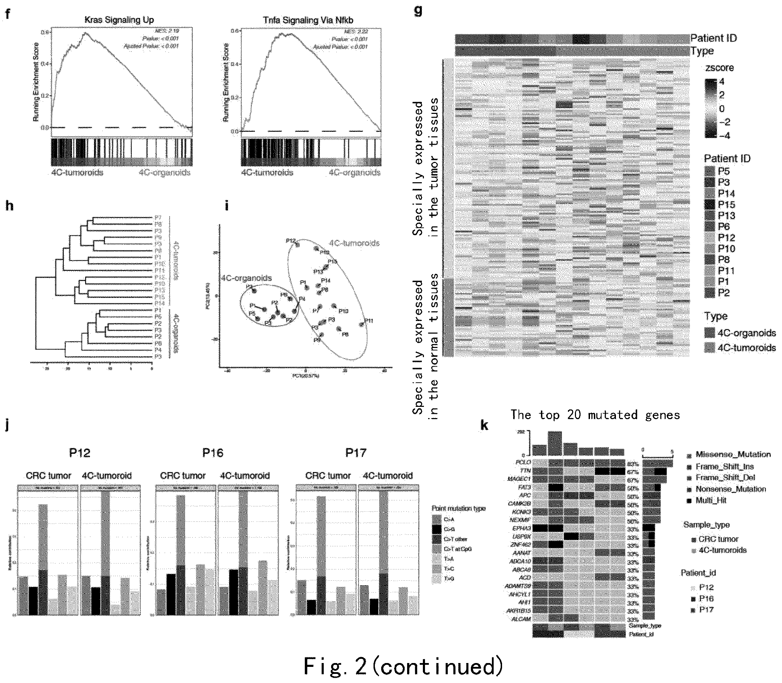

**Figure 2** **shows the establishment of colorectal tumor tissue derived organoids.**
   a. Typical morphology of the human colorectal tumor tissue derived organoids cultured under different conditions. Imaging was performed on day 10 after single-cell passaging.
   b. Quantification of the numbers and sizes of tumor organoids under different conditions (n = 4).
   c. Statistics on of the numbers of tumor organoids under different conditions at different passages (n = 3).
   d. Representative images of the human colorectal tumor tissue derived organoids after the long-term passage (20 passages).
   e. Immunohistochemical and immunofluorescence staining of the renal capsule grafts of colorectal tumor tissue derived organoids and the primary tumor sections of corresponding colorectal tumor patients.
   f. GSEA analysis showing the enrichment of cancer-related gene markers in the colorectal tumor tissue derived organoids, with the human intestinal tissue derived organoids as a control.
   g. Heatmap showing the enrichment of recently reported tumor-specific genes in the colorectal tumor tissue-derivied organoids from different patients. The patient numberings are shown on the right.
   h. Unsupervised hierarchical clustering analysis of the global gene expression profiles of intestinal tissue derived organoids and colorectal tumor tissue derived organoids.
   i. PCA analysis of the expression profiles of intestinal tissue derived organoids and colorectal tumor tissue derived organoids.
   j. Histograms of the proportions of the point mutation types of three colorectal tumor patients (P12, P16, P17) in the colorectal tumor tissue derived organoids and in situ tumors.
   k. Single nucleotide variations (SNVs) heatmap showing the patient-specific mutant genes in the colorectal tumor tissue derived organoids and in situ tumors.

   Abbreviations: Con.: the conventional culture condition of the intestinal organoid; 4C: the novel culture condition of the intestinal organoid; CRC tumor: *in vivo* colorectal tumor tissue.
   *** P < 0.001; ** P < 0.01; ** P < 0.05. Scale bar: 100 µm.

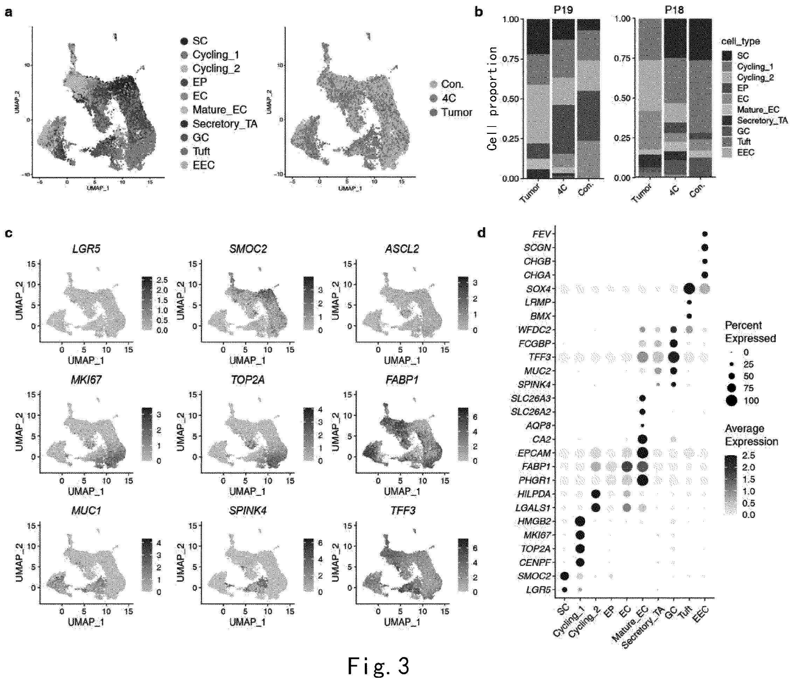

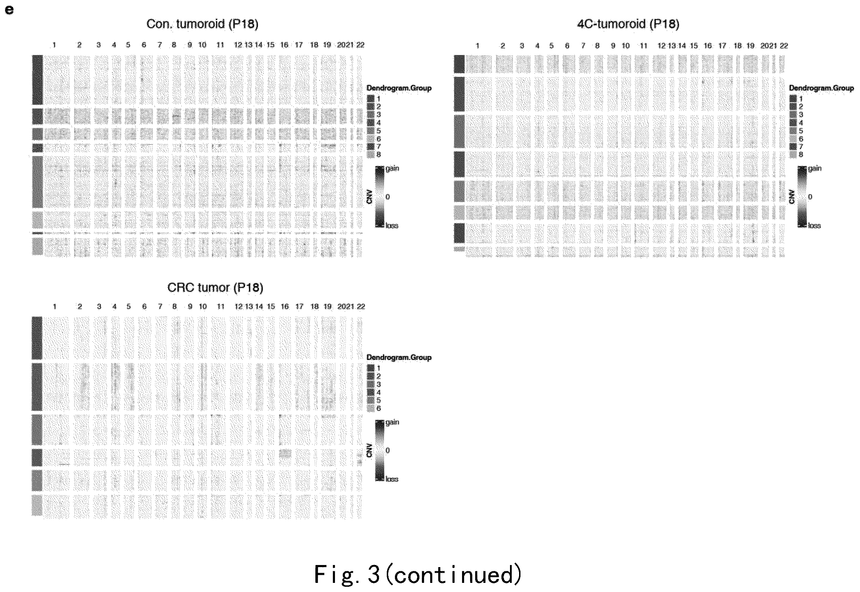

**Figure 3** **shows that the heterogeneity of epithelial cells in the colorectal tumor tissue derived organoids is similar to that of in situ tumors.**
   a. UMAP visualization of the single-cell transcriptome data of the tumor organoids and in situ tumor cells (a total of 20,223 cells) from two patients (P19 and P18). The left panel indicates the result of unsupervised clustering. The right panel indicates the cell sources.
   b. Histograms showing the proportion of each cell type in the tumor organoids and in situ tumors from two patients (P19 and P18).
   c. Representative gene expression of each cell type is superimposed on the UMAP shown in Panel a.
   d. Expression of the characteristic markers for cells of different lineages.
   e. Heatmap showing the large-scale CNVs of the tumor organoids cultured under different conditions and in situ tumors. A total of 8 clusters were identified for each group of cells.

   Abbreviations: Con.: the conventional culture condition of intestinal organoid; 4C: the culture condition of the intestinal organoid of the present invention; tumor: *in vivo* colorectal tumor tissue.
   * * * P < 0.001; ** P < 0.01; ** P < 0.05. Scale bar: 100 µm.
**Figure 4** **shows the establishment of human normal tissue derived organoids.**
   a. Morphology of the organoids obtained by culturing different human normal tissues under the 4C condition.
   b. Immunofluorescence staining of the organoids obtained by culturing different human normal tissues under the 4C condition.
**Figure 5** **shows the establishment of human tumor tissue derived organoids.**
   a. Morphology of the organoids obtained by culturing different human tumor tissues under the 4C condition.
   b. Immunofluorescence staining of the organoids obtained by culturing different human tumor tissues under the 4C condition.
**Figure 6** **shows the establishment of highly deteriorated tumor organoids.**
   a. Representative images of the tumor cells with metastatic and invasive ability passing through matrigel under different conditions in a tumor invasion assay using the colorectal cancer tumor organoids cultured under the 4C condition.
   b. Quantitative statistics of the tumor cells with metastatic and invasive ability under different treatment conditions in Panel a (n=3 wells);
   c. Representative images of the tumor cells with metastatic and invasive ability passing through matrigel under different conditions in a tumor invasion assay using the gastric cancer tumor organoids cultured under the 4C condition.
   d. Quantitative statistics of the tumor cells with metastatic and invasive ability under different treatment conditions in Panel b (n=3 wells);
   e. Representative images of the tumor cells with metastatic and invasive ability passing through matrigel under different conditions in a tumor invasion assay using the lung cancer tumor organoids cultured under the 4C condition.
   f. Quantitative statistics of the tumor cells with metastatic and invasive ability under different treatment condition in Panel e (n=3 wells);
   g. Representative images of the tumor cells with metastatic and invasive ability passing through matrigel under different conditions in a tumor invasion assay using the liver cancer tumor organoids cultured under the 4C condition.
   h. Quantitative statistics of the tumor cells with metastatic and invasive ability under different treatment conditions in Panel g (n=3 wells);
   i. Representative images of tumor cells with metastatic and invasive ability passing through matrigel under different conditions in a tumor invasion assay using the ovarian cancer tumor organoids cultured under the 4C condition.
   j. Quantitative statistics of the tumor cells with metastatic and invasive ability under different treatment conditions in Panel i (n=3 wells);
   k. Flowchart of *in vivo* xenograft experiments in mice with the tumor organoids cultured under the 4C condition.
   l. The colorectal cancer tumor organoids cultured under the 4C condition were transplanted into the spleens of NOG mice after the treatment with WS6 (right) or without WS6 (left). Tumor growth and metastatic spread were monitored by bioluminescence imaging at different weeks after implantation.
   m. The colorectal cancer tumor organoids treated with WS6 (n = 6 mice) were compared with the colorectal cancer tumor organoids not treated with WS6 (n = 6 mice) through the quantification of the number of the formed metastases (left) and the average radioactivity intensity of the formed metastases (right) by the bioluminescence imaging. P-values were calculated using a rank sum test.

Panel n and panel o show the results of Gene Set Variation Analysis (GSVA), wherein the Heatmap (n) and boxplot (o) show the enrichment of epithelial-mesenchymal plasticity-related gene markers in the colorectal cancer tumor organoids treated with or without WS6. The colorectal cancer tumor organoids were derived from 5 patients (P1, P7, P3, P14, P15) (n=15).

* * * P < 0.001; ** P < 0.01; ** P < 0.05. Scale bar: 100 µm.

### DETAILED DESCRIPTION OF THE INVENTION

In order to make the purpose, technical solutions, and advantages of the present invention more clear, the present invention is further described in detail below in conjunction with the specific examples and with reference to the figures.

### DEFINITIONS

As used herein, the term "human tissue organoid" includes a human normal tissue derived organoid or a human tumor tissue derived organoid.

Among them, the human normal tissue derived organoids refer to the three-dimensional cell tissues which are separated from the human normal tissues/organs and self-organized by a high-density accumulation of cells in a controlled space through the three-dimensional culture, each of which highly expresses a marker gene of in situ tissues, for example, the gastric organoids highly express Villin, the lung organoids highly express ACE2, the liver organoids highly express HNF4A, and the ovarian organoids highly express EPCAM.

As used herein, the term "intestinal organoid" refers to a three-dimensional cell tissue that is separated from the human intestinal crypt tissue and self-organized by a high-density accumulation of cells in a controlled space through the three-dimensional culture. In the present invention, the "intestinal organoid" includes the "intestinal tissue derived organoid" derived from the isolated human intestinal crypt tissue and obtained by the 4C culture system of the present invention as described below and the "conventional intestinal organoid" derived from the isolated human intestinal crypt tissue and obtained by a method disclosed by the prior art as described below.

As used herein, the term "intestinal tissue derived organoid" refers to an organoid obtained by isolating the crypt tissue of human intestine and culturing the same under the condition of LDN214117, EGF, CHIR99021, and FGF2 *(i.e.,* the 4C condition) described in the present invention. The "intestinal tissue derived organoid" has the following features: having certain crypt-budding structures in morphology, having very strong amplification and clonogenic abilities, significantly enriching a epithelial cell heterogeneity, such as the activation of goblet cell lineage genes, having a lineage composition similar to that of *in vivo* intestinal crypts, and expressing or significantly highly expressing one or more of the cell marker genes LGR5, OLFM4, CHGA, CHGB, MUC2, AQP8, and LYPD8 for the stemness and differentiation function of in situ intestinal tissue compared with the conventional intestinal organoid.

As used herein, the term "conventional intestinal organoid" refers to an organoid obtained by isolating the crypt tissue of human intestine and culturing the same under the conventional condition disclosed in the piror art (see the section "Experimental Materials and Methods" for details). The "conventional intestinal organoid" has the following features: being spherical in morphology, almost having no crypt-budding structure, and basically only amplifying the stem progenitor cells, and lacking various differentiated lineage cells.

As used herein, the term "tumor organoid" or "tumor tissue derived organoid" refers to an organoid obtained by culturing the tumor cells, such as the colorectal tumor cells. In the present invention, the "tumor organoid" or "tumor tissue derived organoid" includes an organoid derived from the human tumor tissue and obtained by the 4C culture system of the present invention as described below (*e.g*., "the colorectal tumor tissue derived organoid") and "a conventional tumor organoid" derived from the human tumor tissue and obtained by a method disclosed by the prior art as described below, and also includes a malignant tumor organoid (*i.e.*, the tumor organoid with a higher degree of deterioration and a stronger metastatic and invasive ability) obtained by culturing the human tumor tissue or the above-mentioned tumor tissue derived organoid obtained by the 4C culture system of the present invention under the condition of 4C+IKKε inhibitor (*e.g*., WS6).

As used herein, the term "highly deteriorated tumor organoid" or "malignant tumor organoid" refers to an organoid of the tumor cells or tissue that has progressed to clinical stage III/IV, which has a stronger epithelial-mesenchymal plasticity marker gene expression and a stronger metastatic and invasive ability compared to those of stage I/II tumor cells or tissue, such as a significant increase in the expression level of at least one previously reported epithelial-mesenchymal plasticity marker gene (*e.g.*, MSLN, SCEL, LY6D, F3, CCND1, CTSE, *etc.*, see DOI: 10.1126/SCIADV.ABI7640).

As used herein, the term "conventional tumor organoid" refers to an organoid obtained by isolating the human tumor tissue, such as the colorectal tumor tissue, and culturing the same using a conventional condition disclosed by the prior art (see the section "Experimental Materials and Methods" for details).

As used herein, the term "colorectal tumor tissue derived organoid" refers to an organoid obtained by isolating the human colorectal tumor tissue and culturing the same using the 4C condition of the present invention (*e.g*., LDN214117, EGF, CHIR99021, and FGF2). The "colorectal tumor tissue derived organoid" has the following features: having very strong proliferation and clonogenic abilities, having a epithelial cell heterogeneity similar to that of in situ tumor tissus, and expressing or significantly highly expressing one or more of the multi-lineage cell marker genes LGR5, OLFM4, CHGA, CHGB, MUC2, AQP8, and LYPD8 compared with the conventional tumor organoid.

As used herein, the term "matrigel", also known as the extracellular matrix (ECM), consists of a variety of polysaccharides, water, elastin, and glycoproteins. The ECM used in the method of the present invention is provided by Matrigel^{™} (BD Biosciences), which contains laminin, nidogen, and collagen IV.

### Compositions

### (1) ALK inhibitors

Such inhibitors bind to ALK receptors and prevent the binding of cytokines to the corresponding receptors.

Preferred ALK inhibitor includes LDN214117, and the concentration thereof can be used at a conventional concentration, for example, 0.2µM-20µM, preferably 1µM-4µM, and most preferably 2µM. The structural formula thereof is as follows:

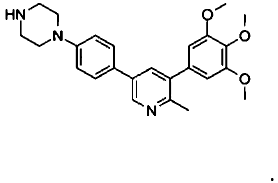

Other ALK inhibitors that are known in the art and commercially available may also be used in the method disclosed herein, including but not limited to: LDN193189, LDN214117, LDN212854, ML347 and K02288.

### (2) EGF agonists

Preferred EGF agonist includes EGF, and the concentration thereof can be used at a conventional concentration, for example, 10 ng/ml-250 ng/ml, preferably 25 ng/ml-100 ng/ml, and most preferably 50 ng/ml.

Other EGF agonists that are known in the art and commercially available may also be used in the method disclosed herein, including but not limited to: EGF, HB-EGF, AREG, EREG, NRG1, NRG2, NRG3 and NRG4.

### (3) WNT agonists

WNT agonists can be used to activate TCF/LEF-mediated transcription in cells.

Preferably, the WNT agonist includes CHIR-99021, and the concentration thereof can be used at a conventional concentration, for example 0.3µM-30µM, preferably 1.5µM-6µM, and most preferably 3µM. The structural formula thereof is as follows:

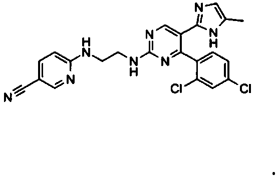

Other WNT agonists that are known in the art and commercially available may also be used in the method disclosed herein, including but not limited to: CHIR-99021, CHIR-98014, CHIR-98023, CHIR-98024, GSK3β inhibitor XV, TD114-2 and BIO-acetoxim.

### (4) FGF agonists

Preferred FGF agonist includes FGF2, and the concentration thereof can be used at a conventional concentration, for example, 10ng/ml-1000 ng/ml, preferably 50 ng/ml-200 ng/ml, and most preferably 100 ng/ml.

Other FGF agonists that are known in the art and commercially available may also be used in the method disclosed herein, including but not limited to: FGF1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21 and FGF23.

### (5) IKKε inhibitors

The concentration of the IKKε inhibitor can be used at a conventional concentration, for example, 0.05µM-5µM, preferably 0.25µM-1µM, and most preferably 0.5µM.

IKKε inhibitors that are known in the prior art and commercially available may be used in the method disclosed herein, including but not limited to: WS3, TBK1/IKKε-IN-5, TBK1/IKKε-IN-2, TBK1/IKKε-IN-6, TBK1/IKKε-IN-4, TBK1/IKKε-IN-1, GSK319347A, HPN-01, AY-985, Amlexanox, MRT67307, BX795, DMX-129, and optionally WS6, wherein the structure of WS6 is as follows:

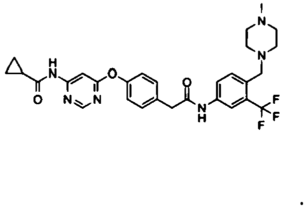

### Examples

### Example 1. Establishment of human intestinal tissue derived organoids.

Through large-scale screening and combination, a new culture condition for human intestinal tissue derived organoids was established, which is obtained by adding 4 components (*i.e.*, 4C; including ALK inhibitors such as LDN214117, EGF agonists such as EGF, WNT agonists such as CHIR-99021, and FGF agonists such as FGF2) to a basal culture medium. Compared with the organoids cultured under the conventional conditions, the human colon organoids cultured under the 4C condition showed a significantly stronger growth rate and an organoid formation ability (Figure 1, a-c). In addition, the human intestinal tissue derived organoids could be amplified for more than 20 generations under the 4C condition and maintain a genomic stability (Figure 1, d-e).

Next, the immunofluorescence staining on the intestinal tissue derived organoids was performed to identify the expression and distribution characteristics of each cell lineage. Through immunofluorescence staining, the markers of multiple lineage cells, including the intestinal stem cell represented by OLFM4, the enteroendocrine cell represented by CHGA, the goblet cell represented by MUC2, and the intestinal epithelial cell represented by APOA1, were observed, all of which were present in the intestinal tissue derived organoids but absent in the conventional intestinal organoids, which was consistent with the previous reports in the literatures (Figure 1, f).

Furthermore, the cell lineage composition in the intestinal tissue derived organoids was analyzed by a single-cell transcriptional profile (scRNA-seq), which was then compared with those in the conventional intestinal organoids and in situ intestinal crypt tissue. To confirm the long-term maintenance of cell heterogeneity, different types of organoids were cultured for at least 2 months. An unsupervised cluster analysis on the organoids and in situ intestinal crypt tissue was performd using the published marker genes of various lineage cells in the human colorectum, obtaining the cluster diagrams of different cell lineages (Figure 1, g-h). Notably, despite the inevitable individual differences, a significant increase in the richness of differentiated cell types was found in the intestinal tissue derived organoids obtained by the system of the present invention compared with that in the conventional intestinal organoids (Figure 1, g-h). Importantly, the lineage composition of the intestinal tissue derived organoids of the present invention was more similar to that of in situ intestinal crypt tissue than that of the conventional intestinal organoids (Figure 1, g, h). Each cell population exhibited different gene expression patterns, which were consistent with those of the corresponding lineages in the human colorectum (Figure 1, i-j). In general, these results indicate that the intestinal tissue derived organoids of the present invention can capture and maintain the cell lineage composition comparable to that of in situ intestinal crypt tissue.

At the same time, different ALK inhibitors were tested, all of which were able to successfully establish the intestinal organoids, with only slight differences in expansion rates, among which LDN-214117 was optimal (Figure 1, k-l).

### Example 2. Establishment of human colorectal tumor tissue derived organoids

Human colorectal tumor tissues were cultured under the 4C condition. Interestingly, despite inevitable individual differences, the human colorectal tumor organoids cultured under the conditions of the present invention (*i*.*e.*, the colorectal tumor tissue derived organoids) consistently showed larger size and higher formation efficiency as well as stronger long-term expansion ability compared with the human colorectal tumor organoids cultured under the conventional conditions (*i.e.*, the conventional tumor organoids) (Figure 2, a-c). In addition, the human colorectal tumor tissue derived organoids of the present invention could be expanded for more than 20 generations under the 4C condition and maintain the typical morphology and expansion ability (Figure 2, d).

Next, the in situ tumor tissue was used as a control to identify the features of the colorectal tumor tissue derived organoids of the present invention. After xenografting the colorectal tumor tissue derived organoids under the kidney capsule of immunodeficient mice, *in vivo* human colorectal tumor tissue was reconstructed. The analysis of histopathological characteristics showed that the xenografts derived from the colorectal tumor tissue derived organoids of the present invention had a similar structure and a tumor marker staining distribution (including Ki-67, CK20, OLMF4, and β-catenin) to those of the corresponding in situ tumor tissue of patient (Figure 2, e). To further study the molecular features of colorectal tumor tissue derived organoids, a global transcriptional profile sequencing (RNA-seq) analysis was performed and the results were compared with those of the intestinal tissue derived organoids. Gene set enrichment analysis further confirmed that colorectal tumor tissue derived organoids were enriched in the tumor-related marker gene groups (Figure 2, f). The recently reported human colorectal tumor marker gene set was used and also found to be highly enriched in the colorectal tumor tissue derived organoids (Figure 2, g). In addition, the dimensionality reduction analysis of the global transcriptional profile showed that the colorectal tumor tissue derived organoids derived from the colorectal tumor tissue had completely different properties from the intestinal tissue derived organoids derived from the intestinal tissue (Figure 2, h-i). In general, these data indicate that the gene expression of the colorectal tumor tissue derived organoids of the present invention is more similar to that of the colorectal tumors.

In order to determine the genomic characteristics of the colorectal tumor tissue derived organoids of the present invention, a whole exome sequencing (WES) was performed, and the gene mutations of the colorectal tumor tissue derived organoids and the corresponding in situ tumor tissue were compared. It was found that the colorectal tumor tissue derived organoids exhibited a point mutation pattern very similar to that of the corresponding in situ tumor (Figure 2, j). The heatmap of gene mutation/variation showed that the characteristic mutant genes of each patient were also reproduced in the colorectal tumor tissue derived organoids (Figure 2, k). In general, these results indicate that the colorectal tumor tissue derived organoids of the present invention can faithfully reflect the histopathological and genomic characteristics of the primary tumor.

Next, a single-cell transcriptional profile (scRNA-seq) analysis was performed to further study the cell lineage composition in the colorectal tumor tissue derived organoids of the present invention, which was then compared with those in the conventional tumor organoids and in situ tumor tissue. To confirm the long-term maintenance of cell heterogeneity, different types of tumor organoids were cultured for at least 2 months. An unsupervised cluster analysis on the tumor organoids and in situ tumor tissue was performed using the published marker genes of various lineage cells in the human colorectum, obtaining the cluster diagrams of different cell lineages (Figure 3, a). Notably, despite the inevitable individual differences, a significant increase in the richness of differentiated cell types was found in the colorectal tumor tissue derived organoids of the present invention compared with that in the conventional tumor organoids (Figure 3, b). For example, the stem cell populations with OLFM4 as a marker gene, the enteroendocrine cells with CHGA and CHGB as marker genes, the goblet cells with MUC2 as a marker gene, and the intestinal epithelial cells with AQP8 and LYPD8 as marker genes, *etc.*, were significantly elevated in the colorectal tumor tissue derived organoids. Importantly, the lineage composition of the colorectal tumor tissue derived organoids of the present invention was more similar to that of in situ tumor epithelial cells than that of the conventional tumor organoids (Figure 3, b). Each cell population exhibited different gene expression patterns, which were consistent with those of the corresponding lineages in human tissues (Figure 3, c-d). In general, these results indicated that the colorectal tumor tissue derived organoids of the present invention can capture and maintain the epithelial heterogeneity comparable to that of in situ tumor.

Considering that the 4C condition can support the culture and expansion of both normal organoids and tumor organoids, the tumor purity in the cultured tumor organoids was estimated to exclude the possibility of normal tissue contamination in the colorectal tumor tissue derived organoids. Based on single-cell transcriptional profiling data, the tumor cells were identified by analyzing the copy number alterations (CNAs) of somatic cells via inferCNV. This analysis can effectively separate the malignant cells with CNAs from the non-malignant cells with normal karyotypes. It was found that the colorectal tumor tissue derived organoids substantially retained the loss and gain of DNA copy number of the corresponding in situ tumor (Figure 3, e). Tumor purity was calculated based on the proportion of malignant cells, and it was found that the proportions of malignant cells in in situ tumor, the conventional tumor organoids, and the colorectal tumor tissue derived organoids of the present invention were 87%, 90%, and 94.2%, respectively (Figure 3, e), indicating that the colorectal tumor tissue derived organoids of the present invention had a high purity of tumor cells. In general, these results indicate that the colorectal tumor tissue derived organoids of the present invention are similar to in situ tumor in terms of the epithelial heterogeneity and tumor purity.

### Example 3. Establishment of human normal tissue derived organoids

The inventors further verified whether the 4C condition of the present invention can be used to culture or obtain the organoids derived from other human tissues, such as a small intestinal tissue derived organoid, a gastric tissue derived organoid, a lung tissue derived organoid, etc.

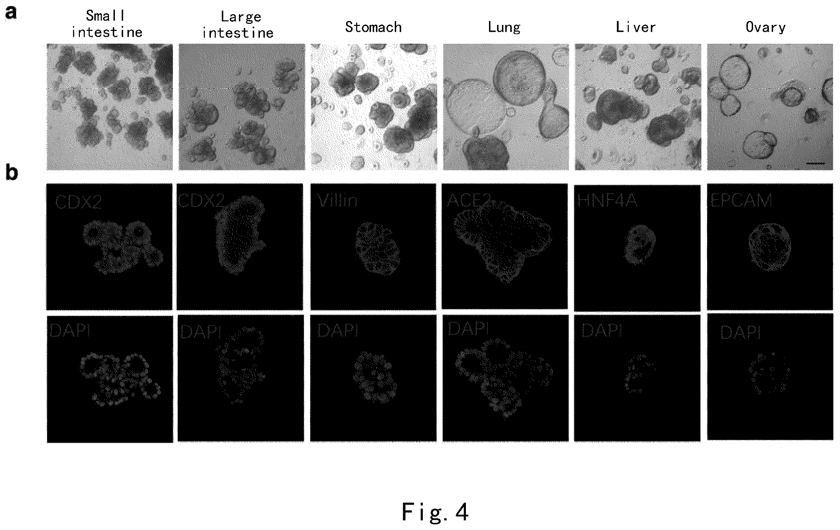

Specifically, referring to the section of "Experimental Materials and Methods", a small intestinal tissue, a large intestinal tissue, a gastric tissue, a lung tissue, a liver tissue and an ovarian tissue were respectively subjected to the tissue separation and the organoid culture (under the 4C culture condition of the present invention) (Figure 4a). Furthermore, the obtained organoids were detected by immunofluorescence staining, and it was found that the human normal tissue organoids cultured under the 4C condition can express the typical genes of the tissues/organs from which they were derived at high levels. Specifically, the intestinal organoids cultured under the 4C condition highly expressed CDX2, the gastric organoids cultured under the 4C condition highly expressed Villin, the lung organoids cultured under the 4C condition highly expressed ACE2, the liver organoids cultured under the 4C condition highly expressed HNF4A, and the ovarian organoids highly expressed EPCAM (Figure 4b). That is, the 4C condition of the present invention can also be used to establish the organoids derived from a variety of human normal tissues, including small intestine, large intestine, stomach, liver, ovary, and lung.

### Example 4. Establishment of human tumor tissue derived organoids

The inventors further verified whether the 4C condition of the present invention can be used to culture or obtain the organoids derived from other human tumor tissues, such as a gastric cancer tumor tissue derived organoid, etc.

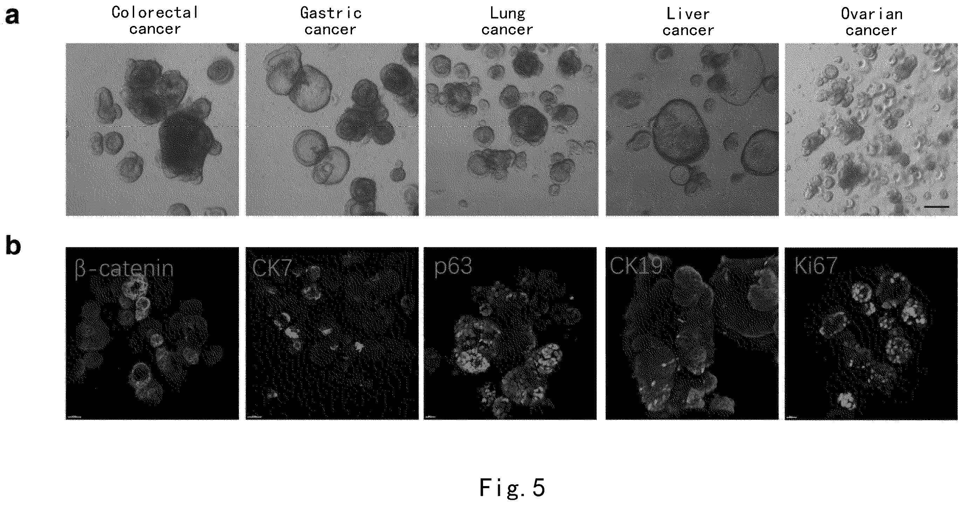

Specifically, referring to the section of "Experimental Materials and Methods", a colorectal cancer tumor tissue, a gastric cancer tumor tissue, a lung cancer tumor tissue, a liver cancer tumor tissue or an ovarian cancer tumor tissue were respectively subjected to the tissue separation and the organoid culture (under the 4C culture condition of the present invention) (Figure 5a). Furthermore, the obtained tumor organoids were detected by immunofluorescence staining, and it was found that the human tumor tissue organoids cultured under the 4C condition can express the typical genes of the tumor tissues from which they were derived at high levels. Specifically, the colorectal cancer organoids cultured under the 4C condition highly expressed β-catenin, the gastric cancer organoids cultured under the 4C condition highly expressed CK7, the lung cancer organoids cultured under the 4C condition highly expressed p63, the liver cancer organoids cultured under the 4C condition highly expressed CK19, and the ovarian cancer organoids cultured under the 4C condition highly expressed Ki67 (Figure 5b). That is, the 4C condition of the present invention can also be used to establish the organoids derived from other cancers/tumors, that is, the corresponding tumor tissue derived organoids can be successfully obtained and cultured under the 4C condition from a variety of tumor tissues (including human colorectal cancer, gastric cancer, lung cancer, liver cancer, and ovarian cancer).

### Example 5. Establishment of tumor organoids with a higher degree of deterioration

Furthermore, the tumor organoids cultured under the 4C condition were used for a large-scale small molecule screening. A series of small molecule IKKε inhibitors were identified, and they can significantly induce the epithelial-mesenchymal morphological transformation of the tumor organoids, wherein said morphological transformation was generally considered to be closely related to the improvement of tumor metastatic and invasive ability. That is, under the condition of 4C + small molecule IKKε inhibitor, the deterioration degree of tumor organoids can be enhanced; alternatively, in situ tumor tissues can be directly cultured under the condition of 4C + small molecule IKKε inhibitor to obtain the tumor organoids with a higher degree of deterioration (*i.e.*, an enhanced metastatic and invasive ability).

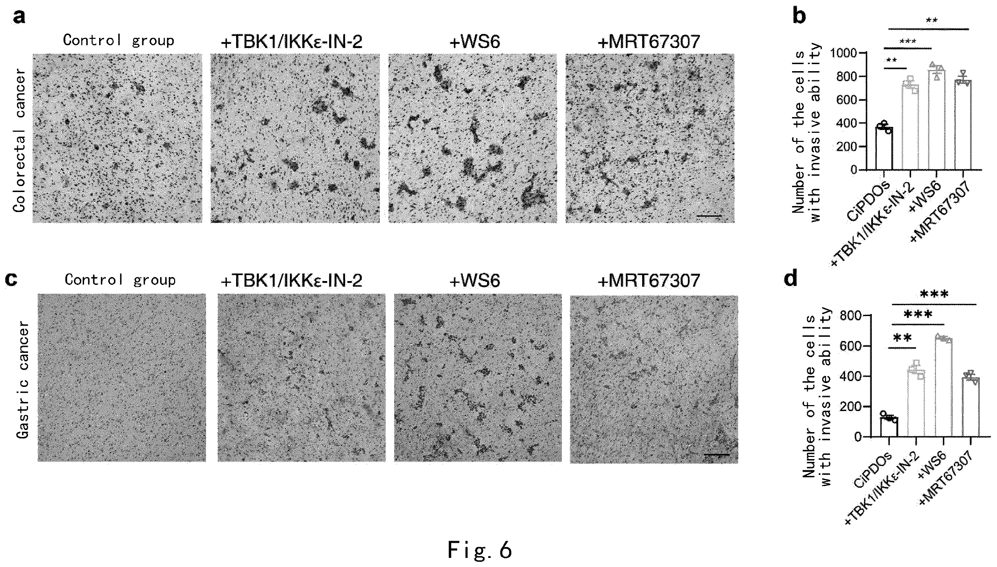

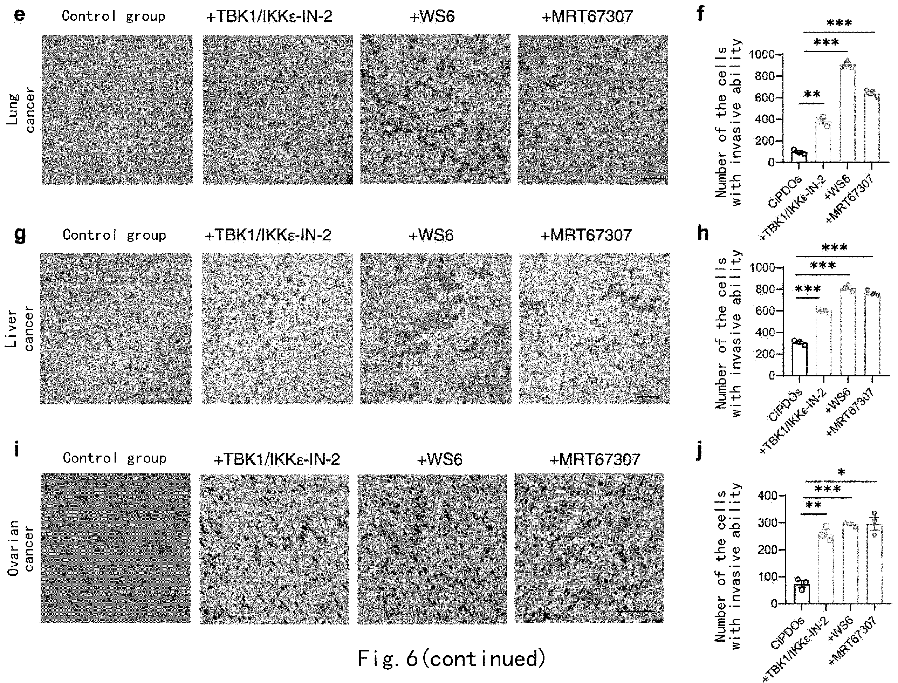

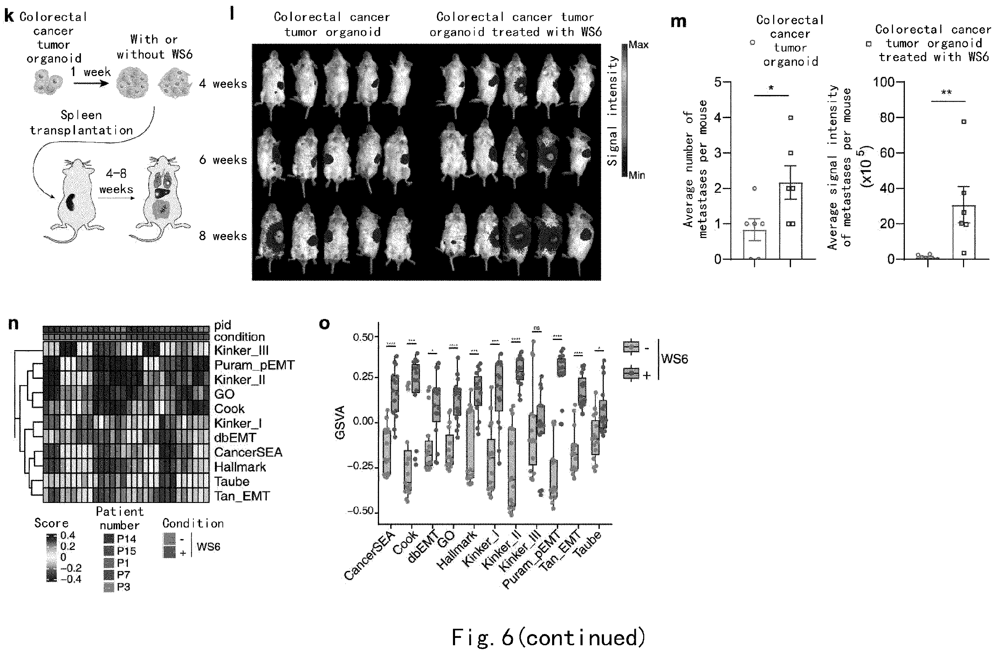

The metastatic and invasive ability of tumor organoids was detected by a permeable cell culture chamber, and it was found that different small molecule IKKε inhibitors, such as TBK1/IKKε-IN-2, WS6 and MRT67307, can significantly enhance the metastatic and invasive ability of tumor organoids dervived from different tumor tissues (Figure 6 a-j). Based on the above results, those skilled in the art can expect that other IKKε inhibitors such as TBK1/IKKε-IN-4, TBK1/IKKε-IN-1, GSK319347A, HPN-01, AY-985, Amlexanox, etc. can also achieve the similar effects. Among these small molecule IKKε inhibitors, WS6 had a relatively stronger effect on the metastatic and invasive ability of tumor organoids. Therefore, the WS6-treated colorectal cancer tumor organoids were transplanted into the immunodeficient mice and the untreated colorectal cancer tumor organoids cultured under the 4C condition were used as a control group. It can be observed that the colorectal cancer tumor organoids treated with WS6 had significantly stronger metastatic and invasive abilities after being transplanted into mice (Figure 6 k-m), which was consistent with the results of the permeable cell culture chamber test.

Furthermore, a global transcriptome analysis was used to compare the colorectal cancer tumor organoids treated with or without WS6. 11 reported epithelial-mesenchymal plasticity gene sets were detected, and the gene set variation analysis (GSVA) showed that 10 of the 11 epithelial-mesenchymal plasticity gene sets were significantly enriched in the tumor organoids treated with WS6 (Figure 6, n-o). These data show that the small molecule IKKε inhibitors can promote the key features of malignant tumors including the enhanced epithelial-mesenchymal plasticity characteristics and metastasis, and that a culture condition containing the small molecule IKKε inhibitor can be used to culture the human tumor organoids with a higher degree of deterioration, which can be used as an *in vitro* model for studying the malignant progression, metastasis or evolution of tumors, or for screening the therapeutic drugs for tumors.

## Claims

1. A composition comprising: (1) an ALK inhibitor, (2) a WNT agonist, (3) an EGF agonist and (4) an FGF agonist; preferably the composition is used to obtain and/or culture a human tissue organoid, for example, a human normal tissue derived organoid or a human tumor tissue derived organoid, such as a human intestinal tissue derived organoid or a human colorectal tumor tissue derived organoid.

2. The composition according to claim 1, wherein the (1) ALK inhibitor is one or more selected from the group consisting of: LDN193189, LDN214117, LDN212854, ML347, and K02288; preferably LDN214117.

3. The composition according to claim 1, wherein the (2) WNT agonist is one or more selected from the group consisting of: CHIR-99021, CHIR-98014, CHIR-98023, CHIR-98024, GSK3β inhibitor XV, TD114-2, and BIO-acetoxim; preferably CHIR-99021.

4. The composition according to claim 1, wherein the (3) EGF agonist is one or more selected from the group consisting of: EGF, HB-EGF, AREG, EREG, NRG1, NRG2, NRG3, and NRG4; preferably EGF.

5. The composition according to claim 1, wherein the (4) FGF agonist is one or more selected from the group consisting of: FGF1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, and FGF23; preferably FGF2.

6. The composition according to any one of claims 1-5, further comprising (5) an IKKε inhibitor;
optionally, the (5) IKKε inhibitor is one or more selected from the group consisting of: WS6, WS3, TBK1/IKKε-IN-5, TBK1/IKKε-IN-2, TBK1/IKKε-IN-6, TBK1/IKKε-IN-4, TBK1/IKKε-IN-1, GSK319347A, HPN-01, AY-985, Amlexanox, MRT67307, BX795, and DMX-129; preferably WS6.

7. A method for obtaining and/or culturing a human tissue organoid, comprising: culturing a human tissue in a culture medium, wherein the culture medium comprises the composition according to any one of claims 1-5; preferably, the human tissue includes a human normal tissue or a human tumor tissue, and the human tissue organoid includes a human normal tissue derived organoid or a human tumor tissue derived organoid.

8. The method according to claim 7, wherein the human normal tissue is a human intestinal tissue, stomach, lung, liver or ovary, and the human tumor tissue is a human colorectal cancer tumor tissue, a human gastric cancer tumor tissue, a human lung cancer tumor tissue, a human liver cancer tumor tissue or a human ovarian cancer tumor tissue.

9. The method according to any one of claims 7-8, wherein DMEM/F12 is used as a basal culture medium;
preferably, HEPES, GlutaMAX, B27, and 1 mM N-Acetylcysteine are further added to the basal culture medium.

10. A human normal tissue derived organoid or human tumor tissue derived organoid obtained by the method of any one of claims 7-9.

11. The human normal tissue derived organoid or the human tumor tissue derived organoid according to claim 10, which is a human intestinal tissue derived organoid or a human colorectal tumor tissue derived organoid, and wherein it expresses one or more of the marker genes LGR5, OLFM4, CHGA, CHGB, MUC2, AQP8 and LYPD8.

12. The human tumor tissue derived organoid of claim 10, for use in *in vitro* identification of the drug resistance of a tumor, the metastatic and invasive ability of a tumor, for use in screening of a drug that is highly effective in inhibiting or killing a tumor cell, or for use as an *in vitro* model for reconstructing the process of tumor development.

13. A method for obtaining and/or culturing a human tumor organoid, comprising: culturing the human tumor tissue or the human tumor tissue derived organoid of claim 10 in a culture medium, wherein the culture medium comprises the composition of claim 6.

14. A human tumor organoid obtained according to the method of claim 13, comprising at least one epithelial-mesenchymal plasticity marker gene with an increased expression level.
